# EUROPEAN PATENT APPLICATION

(11) **EP 1 036 562 A1**
(43) Date of publication of application: **20.09.2000**
(21) Application number: 98953072.0
(22) Date of filing: 16.11.1998
(51) Int. Cl.: A61K 9/16, A61K 9/72, A61K 31/415, A61K 31/395

(54) **SOFT-PELLET DRUG AND PROCESS FOR THE PREPARATION THEREOF**

(30) Priority: 03.12.1997 JP 33322897
(71) Applicant: FUJISAWA PHARMACEUTICAL CO., LTD., Osaka-shi Osaka 541-8514 (JP)
(72) Inventor: YOSHIDA, Hiromitsu, Kyoto-shi, Kyoto 612-0806 (JP); ITO, Hideki, Izumisano-shi, Osaka598-0071 (JP); SOEDA, Yasuko, Takarazuka-shi, Hyogo 665-0015 (JP)
(74) Representative: Gille Hrabal Struck Neidlein Prop Roos
(86) International application number: JP9805159
(87) International publication number: WO9927911

(57) **Abstract**

There provided is a soft pelletized drug composition which is easy to handle in the pharmaceutical manufacturing process, free from the risk for irritable reactions in the user, and controllable in the particle size distribution of the drug to be inhaled. The objective soft pelletized drug composition with a mean particle diameter of 100 µm∼1 mm can be produced by the disclosed technology, for example a method which comprises drying a solution or suspension of the drug substance having self-agglomerative properties in a solution of a sugar such as lactose, micronizing the dry mixture, and rolling the resulting finely micronized powder in a rotary drum.

## Description

### TECHNICAL FIELD

This invention relates to a soft pelletized drug composition and more particularly to a soft pelletized drug composition which is easy to handle in pharmaceutical manufacturing process and easy for the patients to inhale.

### BACKGROUND ART

The prevention or treatment of a disease (e.g. asthma) with an inhalation comprises the delivery of the drug into the airways by the nasal or oral route for attaining a local effect on the bronchi or a systemic effect through pulmonary absorption. It is well known that the inhalation product of dry powder inhaler is formulated with only a finely micronized drug substance, or blended mixture of a finely micronized bulk drug and, for example, a sugar such as lactose, which is filled in a capsule. This capsule shall be set in an inhalation device to be inhaled by user. However, the formulated composition manufactured in the above manner can hardly be controlled in the amount of the drug reaching the target site such as the bronchi or alveolar. Furthermore, in the former case, the finely micronized drug powder is not so flowable that a filing variation is inevitable for the drug contents in the unit capsule because a large portion of the bulk powder tends to stick to the filling machine and the inhalation device and in the inhalation device, there remains much amount of drug undelivered when administered because of their adhesive behavior. Thus, there exist many kinds of problematic matters in the formulation of micronized drug substance. In the latter case, it has been pointed out, for instance, that the larger particle diameter of the sugar formulated with drug substance tends to irritate the patient's pharynx.

Referring to the prior art, Japanese Kokai Tokkyo Koho S48-18420 (corresponding to UK Patent 1381872) discloses an inspirable pharmaceutical composition comprising a simple mixture of a solid drug substance having an effective particle diameter of 0.01∼10 microns and a carrier (an inorganic salt, a saccharide, or the like) having an effective particle diameter of 80∼150 microns. Japanese Kokai Tokkyo Koho S52-83920 and UK Patent 1520247 disclose a soft-pellet comprising agglomerates of individual drug particles at least 90% of which have diameters not greater than 10 microns and which has a particle diameter of 10∼1000 microns and a method of producing the soft pellet. Japanese Kohyo Tokkyo Koho H9-504225 (corresponding to laid-open EP 721332) discloses a method of producing agglomerates of particles or pellets of drug suitable for inhalation. However, those technologies are not fully capable of overcoming the above-mentioned drawbacks.

### DISCLOSURE OF THE INVENTION

Developed in view of the above state of the art, this invention has for its object to provide an inspirable pharmaceutical composition which is easy to handle in its manufacturing process, varying little in delivered dose even if the inhalation rate varies, insuring an efficient production of a finely respirable cloud, and capable of efficient delivery of the drug to the target site such as the bronchi, bronchiole or alveolar.

The inventors of this invention did much research and found that the above-mentioned drawbacks can be neatly obviated with a soft pelletized drug composition prepared, for example, by blending a drug substance having self-agglomerative properties with a sugar in dissolved or suspended state and rolling the resulting mixture in dry state.

The soft pelletized drug composition of this invention comprises a self-agglomerative drug substance and a sugar. More particularly, the composition comprises agglomerates of a drug substance particles having self-agglomerative properties and a sugar particles. The term "drug substance having self-agglomerative properties" as used throughout this specification means a medicinally active compound such that its bulk substance itself has self-agglomerative properties or that its finely micronized powder has self-agglomerative properties. The term "soft pelletized drug composition" is used in this specification to mean a pharmaceutical composition which has a mean particle diameter of about 100 µm∼1 mm and is not broken under the usual conditions of handling processes such as weighing and loading into an inhalation device but is easily broken up into the required particle size within the inhalation device. The inhaler of this invention uses the above soft pelletized drug composition.

The method of manufacturing a soft pelletized drug composition according to the invention comprises dissolving or suspending a drug substance having self-agglomerative properties in a sugar solution, drying the resulting solution or suspension, micronizing the dried mixture, and rolling the micronized powder mixture. As an alternative, the soft pelletized drug composition of the invention can also be obtained by micronizing a homogeneous mixture of a drug substance having self-agglomerative properties and a sugar or blending a finely micronized powder of said drug substance evenly with a sugar and rolling the mixture.

### BEST MODE FOR CARRYING OUT THE INVENTION

The embodiments of the invention are now described. The drug substance for the soft pelletized drug composition of this invention is a compound having self-agglomerative properties and, as such, includes but is not limited to disodium cromoglycate, tacrolimus (FK-506), budesonide, terbutaline sulfate, isoproterenol sulfate, beclomethasone dipropionate, salbutamol, and compounds having neuroquinine antagonist activity as disclosed in Kokai Tokkyo Koho H4-210996, such as N²-[(4R)-4-hydroxy-1-[(1-methyl-1H-indol-3-yl)carbonyl]-L-prolyl]-N-benzyl-N-methyl-3-(2-naphthyl)-L-alaninamide (hereinafter referred to as Compound A). Compound A is represented by the following chemical formula.

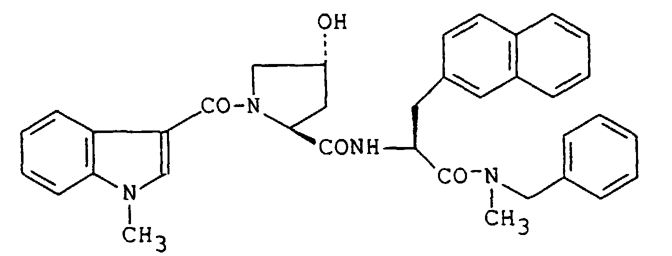

The sugar for this invention includes but is not limited to glucose, galactose, and fructose, among other monosaccharides, and lactose, sucrose, and maltose, among other disaccharides. Particularly preferred is lactose. The ratio, by weight, of the drug substance to the sugar may be varied within the range of 100:1∼1:100, preferably 10:1∼1:10, and more preferably 5:1∼1:5.

Depending on the solubility characteristic of the drug substance used, the invented manufacture method of the soft pelletized drug composition may for example comprise either dissolving the sugar in water and mixing the solution with a solution of the drug substance in ethanol or the like or dissolving the sugar in water and dissolving or suspending the drug substance therein, followed by drying the resulting solution or suspension by the vacuum drying technique or the like, micronizing it in a milling machine such as a jet mill to a particle diameter of not greater than 10 µm, feeding the resulting powder into a rotary drum, and rolling it at a drum speed of 10 rpm∼50 rpm for a powder residence time of 20∼60 minutes at room temperature. By this rolling procedure, the particles of said drug substance and the particles of sugar form together agglomeraes to give a soft pelletized drug composition having a suitable particle diameter. An alternative procedure comprises blending a drug substance having a particle diameter of about 100 µm evenly with a sugar, micronizing the resulting mixture to a mean particle diameter of not greater than 10 µm, feeding the micronized powder to a rotary drum, and thence proceeding in the same manner as described above to provide the objective soft pelletized drug composition. A further alternative procedure comprises micronizing the drug substance to a particle diameter of not greater than 10 µm, blending the resulting powder evenly with a sugar, feeding the resulting mixture to a rotary drum, and thence proceeding in the same manner as described above to provide the objective soft pelletized drug composition.

The mean particle diameter of the soft pelletized drug composition, thus obtained, of the invention lies within the range of about 100 µm to 1 mm but may be adjusted according to the performance characteristics of the inhalation device. The preferred mean particle diameter is 150 µm∼350 µm. The thus-obtained soft pelletized drug composition of the invention is filled in a cartridge such as a hard capsule which is set in an inhalation device for administration. In the case of Compound A which has high self-agglomerative properties, it was found that a soft pelletized composition having a mean particle diameter of about 100 µm∼1 mm, which is easy to handle and has excellent respirable characteristics, can be obtained even without the aid of a sugar by the simple procedure of micronizing its bulk substance and rolling the resulting fine powder in a rotary drum under the above-mentioned conditions. This product is preferable for patients who are prone to receive irritable responses to larger sugars particles.

### EXAMPLES

The following examples illustrate this invention.

### Example 1

In 144 g of absolute ethanol was dissolved 5 g of Compound A. Separately, 5 g of lactose was dissolved in 96 g of water to prepare an aqueous solution of lactose. The two solutions were blended to give 250 g of a mixed solution. This mixed solution was dried in vacuo at 40°C to give a dry mixture. This dry mixture of Compound A and lactose was comminuted and sieved through a 850 µm screen. The finely divided powder of Compound A thus obtained was fed to a rotary drum which was rotated at the drum speed of 37 rpm at room temperature for 30 minutes. The resulting product was then subjected to size selection to provide a soft pelletized drug composition comprising Compound A and lactose and having a mean particle diameter of 180 µm∼350 µm. The weight ratio of Compound A to lactose was controlled to be 1:1.

### Example 2

A dried lot of Compound A was pulverized and sieved through a 850 µm screen. The resulting powder, which was exclusively composed of Compound A, was fed to a rotary drum and further processed as in Example 1 to provide a soft pelletized drug preparation composed exclusively of Compound A. The soft pelletized drug preparation thus obtained was filled into a hard capsule and by using an inhalation device its inspiration characteristics were investigated. The results are shown in Table 1.

**Table 1**

| Inhalation characteristics of soft pelletized drug preparations | | | | |
|---|---|---|---|---|
| Preparation | With lactose | | Compound A alone | |
| Compound A content | 10 mg | 20 mg | 10 mg | 20 mg |
| Emitted Dosee (%) | 63.6 | 50.6 | 78.4 | 62.4 |
| Respirable Fraction (%) | 60.6 | 72.0 | 39.6 | 49.9 |
| Delivered dose (%) | 38.3(3.83 mg) | 36.5(7.29 mg) | 31.0(3.10 mg) | 30.7(6.14 mg) |

Inhalation rate: 28.3 liters/min.
Measuring instrument: Dylec cascade impactor (with a glass throat).
Inhalation device: E-haler
Respirable fraction: The percentage of Compound A particles less than 6.4 µm in diameter in the emitted dose of Compound A (fine particle fraction).
Delivered dose: The percentage of delivered Compound A particles less than 6.4 µm in diameter relative to the Compound A content (e.g. 10 mg) (fine particle dose).

The flowability of the invented soft, pelletized drug composition shown in Table 2.

A powder tester was used for determination and each index was calculated by the method proposed by Carr. The Carr method is described in Chemical Engineering, Vol. 18, Jan., 166-167 (1965).

**Table 2**

| Flowability of the soft pellets of the invention | | | | |
|---|---|---|---|---|
| Sample | [I] Index | [II] Index | [III] Index | [IV] Index |
| Angle of repose | 12 | 12 | 16 | 2 |
| Compressibility | 0 | 0 | 0 | 17 |
| Angle of Spatula | 12 | 12 | 12 | 17.5 |
| Uniformity | 23 | 25 | 23 | 23 |
| Flowability index | 47 | 49 | 51 | 78.5 |
| Notes [I]: Lactose JP (Japanese Pharmacopoeia) [II]: A mixture of [I] and [III] [III]: A micronized powder of Compound A [IV]: The soft pellet of the invention (with lactose) | | | | |

It is apparent from the above data that because the self-agglomerative properties of Compound A were lessened by the addition of the lactose, the soft pelletized composition of the invention was definitely increased in respirable fraction. It is also clear that the flowability of the soft pelletized composition of the invention is definitely superior to that of a simple mixture of Compound A and lactose. Whereas the mean particle diameter of the same finely micronized powder of Compound A (containing lactose) as above prior to processing into soft pellets was 2.5 µm, the mean particle diameter of the soft pellets was 308 µm (method of measurement: sieving, classification time: 3 min.). Soft pellets of this size are very easy to handle in weighing and cartridge loading. Moreover, as the above inspiration characteristic data indicate, the soft pelletized composition of the invention yields a sufficiently fine cloud upon delivery to be suited for inhalation, thus overcoming the disadvantages of the prior art.

### Example 3

Using the soft pellets composed exclusively of Compound A and the soft pellets composed of Compound A and lactose, a comparison in delivered dose was made by varying the inhalation rate with the cascade impactor. The soft pellets of the invention were prepared by blending a finely micronized powder of Compound A evenly with lactose, sieving the mixture, rolling it in a rotary drum, and subjecting the product to size selection. The soft pellets composed exclusively of Compound A were prepared in the same manner except that lactose was not added. The results of measurement are shown in Table 3.

**Table 3**

| Delivered dose at varying inhalation rate | | | | |
|---|---|---|---|---|
| | 40 mg Capsule of Compound A alone | | 40 mg Capsule with lactose | |
| Inhalation rate | 28.3 | 60 | 28.3 | 60 |
| Delivered dose | 9.5 | 16.3 | 11.6 | 11.1 |

40 mg Capsule with lactose: Compound A 40 mg and lactose 13.3 mg are contained.
Inhalation device: E-haler
Inhalation rate: L/min.
Delivered dose (the amount of drug particles with diameters less than 5.8 µm in the cloud): mg

As obvious from the above data, the pharmaceutical ingenuity of this invention, that is addition of a sugar, relieves the intake amount of the drug of the usual dependence on inhalation rate and insures dosing with a minimum of variation. Accordingly, a medicament in soft pellet form of this invention is tailored one to a patient independently on his gender and age and the severity of illness.

### Example 4

1 g of tacrolimus(FK506) was evenly blended with 99 g of lactose and the mixture was pulverized and sieved through a 500 µm screen. The finely micronized powder obtained was fed to a rotary drum which was rotated at 20 rpm at room temperature for 30 minutes. The product was subjected to size selection to provide a soft pelletized drug composition with a mean particle diameter of 180 µm∼600 µm containing tacrolimus and lactose in a weight ratio of 1:99.

### Industrial Applicability

The soft pelletized drug composition provided in accordance with this invention has a low bulk density as compared with a simple mixture of the drug substance and lactose prepared by the conventional technology so that it can be easily handled at loading into the inhalation device. Moreover, it has the advantage that the pellets are broken up in the inhalation device, for example E-haler (made by Rhone-Poulene Rorer), to a particle size range suitable for inhalation at dosing.

Furthermore, in the soft pelletized drug composition of the invention, the particle size distribution of the drug upon inhalation can be controlled by varying the amount of addition of a sugar to a drug substance. In addition, when the proportions of the sugar and drug substance are varied, pellets varying in the concentration of the drug substance but little altered in specific volume can be manufactured. The consequent advantage, which cannot be overlooked, is that the specifications of the filling machine and the inhalation device need not be modified even when drug content is smaller. Another advantage is that even if the drug substance is water-insoluble, the sugar dissolves in water so that the drug substance remained in the device after dosing can be efficiently scavenged by washing with water. It is also noteworthy that owing to the pharmaceutical ingenuity of formulating a sugar with a drug substance, the inspiration amount of the drug is no longer dependent on inhalation rate so that medication with a minimum of dose variation can be insured independently on the patient^{'}s age and gender and the severity of illness.

## Claims

1. A soft pelletized drug composition comprising a drug substance having self-agglomerative properties and a sugar.

2. The soft pelletized drug composition according to Claim 1 wherein the drug substance is a member selected from the group consisting of N²-[(4R)-4-hydroxy-1-[(1-methyl-1H-indol-3-yl)carbonyl]-L-prolyl]-N-benzyl-N-methyl-3-(2-naphthyl)-L-alaninamide and tacrolimus.

3. The soft pelletized drug composition according to Claim 1 or Claim 2 wherein the sugar is lactose.

4. The soft pelletized drug composition according to Claim 1 to Claim 3 wherein the drug substance and sugar are formulated in a weight ratio of 100:1∼1:100.

5. The soft pelletized drug composition according to Claim 1 to Claim 4 which has a mean particle diameter of 100 µm∼1 mm.

6. An inhaler using the soft pelletized drug composition claimed in Claim 1 to Claim 5.

7. A method of manufacturing a soft pelletized drug composition which comprises dissolving or suspending a drug substance having self-agglomerative properties in a solution of a sugar, drying the solution or suspension to give a dry mixture, micronizing the dry mixture, and rolling the resulting finely micronized powder.

8. A method of producing a soft pelletized drug composition which comprises rolling a finely divided homogeneous mixture of a drug substance having self-agglomerative properties and a sugar or a homogeneous mixture of a finely micronized drug substance and a sugar.

9. A soft pelletized drug composition having a mean particle diameter of 100 µm∼1 mm which is available upon rolling a finely micronized powder of N²-[(4R)-4-hydroxy-1-[(1-methyl-1H-indol-3-yl)carbonyl]-L-prolyl]-N-benzyl-N-methyl-3-(2-naphthyl)-L-alaninamide or tacrolimus.

10. An inhaler using the soft pelletized drug composition claimed in Claim 9.

11. A method of manufacturing a soft pelletized drug composition having a mean particle diameter of 100 µm∼1 mm which comprises pulverizing N²-[(4R)-4-hydroxy-1-[(1-methyl-1H-indol-3-yl)carbonyl]-L-prolyl]-N-benzyl-N-methyl-3-(2-naphthyl)-L-alaninamide or tacrolimus and rolling the resulting finely micronized powder.
